# EUROPÄISCHE PATENTSCHRIFT

(11) **EP 0 596 395 B1**
(45) Veröffentlichungstag und Bekanntmachung des Hinweises auf die Patenterteilung: **28.06.2000**
(21) Anmeldenummer: 93117399.1
(22) Anmeldetag: 27.10.1993
(51) Int. Cl.: C07C 45/63, C07C 49/813, C07B 39/00

(54) **Verfahren zur Herstellung fluorierter Benzile**
Process for the preparation of fluorinated benzil
Procédé pour la préparation de benziles fluorées

(30) Priorität: 04.11.1992 DE 4237200
(43) Veröffentlichungstag der Anmeldung: 11.05.1994
(73) Patentinhaber: Clariant GmbH, 65929 Frankfurt am Main (DE)
(72) Erfinder: Kanschik-Conradsen, Andreas, Dr., D-30826 Garbsen (DE); Papenfuhs, Theodor, Dr., D-60433 Frankfurt am Main (DE)

(56) Entgegenhaltungen:
- EP-A- 0 214 565
- GB-A- 1 265 052
- US-A- 4 453 009
- CHEMICAL ABSTRACTS, vol. 076, no. 9, 28. Februar 1972, Columbus, Ohio, US; abstract no. 041832, LALEZARI I ET AL 'Synthesis and pharmacological activity of dialkylaminoalkyl esters of benzilic acids containing fluorine or trifluoromethyl groups' & J. MED. CHEM. (JMCMAR);71; VOL.14 (11); PP.1138-40 UNIV. TEHRAN;FAC. PHARM.; TEHRAN; IRAN
- CHEMICAL ABSTRACTS, vol. 117, no. 23, 7. Dezember 1992, Columbus, Ohio, US; abstract no. 233927, BULMAN PAGE P C ET AL 'A convenient preparation of symmetrical and unsymmetrical 1,2-diketones: application to fluorinated phenytoin synthesis' & TETRAHEDRON (TETRAB,00404020);92; VOL.48 (35); PP.7265-74 UNIV. LIVERPOOL;DEP. CHEM.; LIVERPOOL; L69 3BX; UK (GB)
- CHEMICAL ABSTRACTS, vol. 077, no. 23, 4. Dezember 1972, Columbus, Ohio, US; abstract no. 151617, CHAMBERS R D ET AL 'Polyfluoroaryl organometallic compounds. XV. Synthesis and rearrangement of polyhaloaryl.alpha.-diketones' & J. CHEM. SOC., PERKIN TRANS. 1 (JCPRB4);72; (19); PP.2464-9 UNIV. DURHAM;DEP. CHEM.; DURHAM; ENGL.

## Beschreibung

Gegenstand der vorliegenden Erfindung ist ein neues, vorteilhaftes Verfahren zur Herstellung von fluorierten Benzilen durch Halex-(Chlor-Fluor)-Austauschreaktion aus den entsprechenden chlorierten Verbindungen.

Fluorhaltige Benzile sind Zwischenprodukte zur Herstellung von pharmazeutischen Wirkstoffen. So dient beispielsweise 4,4'-Difluorbenzil zur Herstellung entzündungshemmender Verbindungen (US 4 008 232). Weiterhin lassen sich Benzile durch Spaltung der zentralen C-C-Bindung, z. B. durch Oxidation mit Luftsauerstoff (DE-OS 2 732 149), in zwei gegebenenfalls unterschiedliche Benzoesäuren überführen, deren eine mindestens ein im Sinne des erfindungsgemäßen Verfahrens eingeführtes Fluoratom enthält. Auf diese Weise kann man zum Beispiel zu wertvollen, als Vorprodukte für antibakterielle Mittel (EOS 227 088; DE-OS 3 600 891; DE-OS 3 420 743; EOS 191 185; JP 60 072 885; J. Med. Chem. 31, (1988), 983 - 991) dienenden Trihalogenbenzoesäuren, wie 2-Chlor-4,5-difluorbenzoesäure oder 2,4,5-Trifluorbenzoesäure, gelangen.

Solche Verbindungen wurden bisher auf verschiedenen Wegen aus bereits fluorhaltigen Ausgangsverbindungen hergestellt. So kann beispielsweise 2,2'-Difluorbenzil durch Benzoinkondensation von 2-Fluorbenzaldehyd und anschließende Oxidation hergestellt werden (J. Org. Chem. 23, 1958, 1539 - 1541). Die Verwendung von fluorierten Benzaldehyden macht diesen Syntheseweg unwirtschaftlich und daher unattraktiv. 4,4'-Difluorbenzil ist beispielsweise in einer fünfstufigen Synthese aus Fluorbenzol und Chloral zugänglich (Helv. Chim. Acta 38 (1955), 46, 66; J. Org. Chem. 23 (1958), 1306; J. Am. Chem. Soc. 69 (1947) 667). Dieser Syntheseweg ist aufgrund der Vielzahl der Stufen, der Verwendung von Cyaniden, sowie durch Einsatz von je nach Marktlage ungünstigem und/oder schwer verfügbarem Fluorbenzol als Ausgangsmaterial kostspielig und ungeeignet.

Es bestand daher ein erheblicher Bedarf an einem günstigeren Syntheseweg.

Halex-(Chlor-Fluor)-Austauschreaktionen an chlorierten Benzaldehyden sind seit einigen Jahren bekannt (DE-OS 3 637 156). Auch die Herstellung fluorierter Benzophenone, beispielsweise des 4,4'-Difluorbenzophenons, durch Halex-Reaktion ist prinzipiell bekannt (EP 101 760). Die Herstellung von fluorierten Benzilen durch dieses Syntheseprinzip wurde dagegen bisher noch nicht beschrieben. Die hierfür möglicherweise wesentlichen Gründe werden nachstehend kurz beschrieben. Aus der Literatur ist seit langem bekannt, daß sich aromatische α-Diketone bei der Behandlung mit Basen zu α-Hydroxycarbonsäuren umlagern (Benzil-Benzilsäure Umlagerung, Advanced Organic Chemistry, J. March, 3rd Ed., J. Wiley ed., N. Y. (1985), 969). Da aber Alkalimetallfluoride in aprotischen Systemen recht starke Basen sind (Yakobson et al., Synthesis (1983), 169), bestand durchaus die Möglichkeit, daß die Umsetzung, beispielsweise von
4,4'-Dichlorbenzil mit einem Alkalimetallfluorid, nicht zum gewünschten 4,4'-Difluorbenzil, sondern zumindest teilweise durch Nebenreaktion zum 4,4'-Dichlorbenzilsäurefluorid bzw. dessen Folgeprodukten führt.

Es wurde nun überraschenderweise gefunden, daß man Benzile der allgemeinen Formel 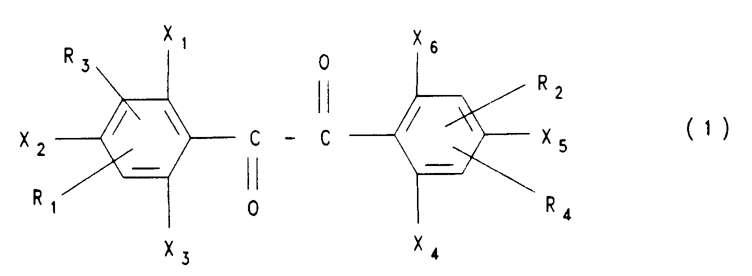 in welcher X₁, X₂, X₃, X₄, X₅, X₆, R₁, R₂, R₃ und R₄ Wasserstoff-, Fluor-, Chlor- oder Bromatome, Alkyl(C₁-C₁₀)-gruppen, Alkoxy(C₁-C₄)-gruppen, gegebenenfalls substituierte Arylgruppen, wie beispielsweise Phenyl- oder Naphthylgruppen, die durch Fluor-, Chlor- oder Bromatome, Alkyl(C₁-C₄)-, Alkoxy(C₁-C₄)-, Nitro-, Cyano-, -CHO-, -COCl-, -COF-, CF₃-, -SO₂-Alkyl(C₁-C₄)-, -SO₂F-, -SO₂Cl-, -COO-Alkyl(C₁-C₄)-, -CO-N(Alkyl(C₁-C₄))₂-, -CO-Phenyl-, -SO₂-Phenyl-gruppen substituiert sein können, oder die Elektronendichte herabsetzende Gruppen, wie beispielsweise Nitro-, Cyano-, - CHO-, -COCl, -COF, -CF₃, -SO₂-Alkyl(C₁-C₄)-, -SO₂F-, -SO₂Cl-, -CON(Alkyl(C₁-C₄))₂, -CO-Phenyl-COO-Alkyl(C₁-C₄)-, -CO-Phenyl oder -SO₂Phenyl-gruppen bedeuten, mit der Maßgabe, daß mindestens einer der Substituenten X₁ - X₆ ein Fluoratom darstellt, in vorteilhafter Weise herstellen kann, in dem man 1 Mol eines Benzils der genannten allgemeinen Formel (1), in welcher X₁ - X₆ und R₁, R₂, R₃ und R₄ die genannten Bedeutungen haben, mit der Maßgabe, daß mindestens einer der Substituenten X₁ - X₆ ein Chloratom darstellt, mit 0,8 bis 2,5 mol Kalium-, Rubidium-, Cäsium- oder Tetraalkyl(C₁-C₁₈)-ammoniumfluorid oder Mischungen davon pro auszutauschendem Chloratom bei Temperaturen von 100 ° bis 280 °C, vorzugsweise von 160 ° bis 240 °C, in Abwesenheit oder in Gegenwart eines Phasentransferkatalysators und in Abwesenheit oder in Gegenwart eines dipolar aprotischen oder unpolaren Lösungsmittels umsetzt.

Als dipolar aprotische Lösungsmittel können beim erfindungsgemäßen Verfahren Sulfolan (Tetramethylensulfon), Tetramethylensulfoxid (TMSO), N,N-Diethylacetamid, N,N-Dimethylacetamid (DMAc), N,N-Dimethylformamid (DMF), N-Methylpyrrolidon (NMP), Dimethylsulfoxid (DMSO), Dimethylsulfon, Diphenylsulfoxid, Diphenylsulfon, Tetramethylharnstoff, Tetra-n-butylharnstoff, 1,3-Dimethylimidazolidin-2-on (DMI) oder Mischungen daraus angewandt werden.

Geeignete unpolare Lösungsmittel sind beispielsweise Chlornaphthalin, Dichlorbenzol, Toluol oder Xylole.

An Fluoridsalzen werden Kalium-, Rubidium-, Cäsium- oder Tetraalkyl(C₁-C₁₈)-ammoniumfluorid oder Mischungen daraus in Mengen von 0,8 bis 2,5 Mol Fluorid pro auszutauschendem Chloratom, bevorzugt 0,9 bis 1,5 Mol, besonders bevorzugt 1,0 bis 1,2 Mol eingesetzt. Kaliumfluorid sowie Mischungen aus Kaliumfluorid und Cäsiumfluorid sind bevorzugt; besonders bevorzugt ist Kaliumfluorid. Das erfindungsgemäße Verfahren läßt die Verwendung von sprühgetrocknetem Fluoridsalz zu, was aber zum Erhalt guter Ergebnisse nicht erforderlich ist.

Das erfindungsgemäße Verfahren kann sowohl mit als auch ohne Zusatz eines Phasentransferkatalysators durchgeführt werden. Durch Verwendung von Phasentransferkatalysatoren gelingt aber in einigen Fällen eine nahezu quantitative und erheblich beschleunigte Umsetzung. Als Phasentransferkatalysatoren kommen quartäre Ammonium- oder Phosphoniumverbindungen wie Tetraalkyl(C₁-C₁₈)-ammoniumchloride, -bromide oder -fluoride, Tetraalkyl(C₁-C₁₈)-phosphoniumchloride oder -bromide, Tetraphenylphosphoniumchlorid oder -bromid, ((Phenyl)ₘ(alkyl(C₁-C₁₈)ₙ)-phosphoniumchloride oder -bromide, wobei m = 1 bis 3, n = 3 bis 1 und m + n = 4 sind, ferner Kronenether, wie zum Beispiel 18-Krone-6, und Dialkylaminopyridiniumsalze oder Mischungen dieser Verbindungen in Frage. Diese Stoffe werden in Mengen von 0,01 bis 50 Molprozent eingesetzt, bevorzugt zwischen 0,5 bis 10 Molprozent, besonders bevorzugt zwischen 1 und 5 Molprozent, jeweils bezogen auf zu fluorierendes Benzil. Verwendet man Tetraalkyl(C₁-C₁₈)-ammoniumfluorid als Fluoridsalz, so ist der gesonderte Zusatz eines Phasentransferkatalysators nicht erforderlich, weil das Fluoridsalz selbst bereits einen solchen Katalysator darstellt und somit in stöchiometrischen oder größeren Mengen eingesetzt werden kann.

Ferner können Oligo- oder Polyethylenglykoldimethylether als Phasentransferkatalysatoren verwendet werden. Die Zahl der Glykoleinheiten in diesen Verbindungen kann von n = 4 (Tetraethylenglykoldimethylether) bis n = 150 betragen, bevorzugt werden jedoch Ether eingesetzt, deren Polymerisationsgrad zwischen n = 4 und n = 25 beträgt. Die optimale Einsatzmenge dieser Glykolether liegt zwischen 0,5 Massenprozent und 200 Massenprozent, bevorzugt zwischen 5 und 100 Massenprozent, besonders bevorzugt zwischen 10 und 50 Massenprozent, jeweils bezogen auf die Masse des eingesetzten Fluoridsalzes. Der besondere Vorteil bei der Verwendung dieser Verbindungen liegt darin, daß meist der Einsatzmenge entsprechend weniger Lösungsmittel verwendet werden kann, weil die Glykolether bei der Reaktionstemperatur stets flüssig sind.

Es ist weiterhin möglich, Mischungen aus diesen Ammonium-, Phosphonium-, Pyridiniumverbindungen, Kronenethern und Glykolethern einzusetzen.

Für den Fall, daß feuchte Ausgangsmaterialien eingesetzt werden, kann die Reaktionsmischung durch teilweises Abdestillieren einer Komponente - die einen höheren Siedepunkt als Wasser besitzt - getrocknet werden. Diese Trocknung kann auch durch Abdestillieren einer Komponente - die ein Azeotrop mit Wasser bildet - erreicht werden. Wenn die Ausgangsmaterialien nur geringe Mengen an Wasser enthalten oder wenn leicht flüchtige Zersetzungsprodukte während der Reaktion entstehen, können diese durch Zusatz eines Lösungsmittels, dessen Siedepunkt unterhalb dem des Produktes liegt, entfernt werden. Hierzu wird dann während der Reaktion dieses Lösungsmittel zum Beispiel über einen Wasserabscheider aus der Reaktionsmischung abdestilliert.

Das Verfahren kann sowohl bei Atmosphärendruck als auch bei Unter- oder Überdruck durchgeführt werden.

Das erfindungsgemäße Verfahren ist besonders dazu geeignet, die bisher noch nicht beschriebenen fluorierten Benzile 2,2',4,4'-Tetrafluorbenzil und 3,3'-Dichlor-4,4'-difluorbenzil aus dem entsprechenden 2,2',4,4'-Tetrachlorbenzil bzw. 3,3',4,4'-Tetrachlorbenzil durch Chlor-Fluor-Austauschreaktion herzustellen.

Das erfindungsgemäße Verfahren ist nicht auf die Umsetzung von symmetrischen Benzilen beschränkt; unsymmetrische Benzile können analog fluoriert werden.

Benzile, die unter die genannte allgemeine Formel (1) fallen und gleichzeitig als neu anzusehen sind, sind unter anderem 2-Chlor-4,5-difluorbenzil, 2,2'-Dichlor-4,4',5,5'-tetrafluorbenzil, 2,2',4,4',5,5'-Hexafluorbenzil, 2,4,5-Trifluorbenzil. Die verfahrensgemäß eingesetzten symmetrischen Ausgangsmaterialien können, wie literaturbekannt (J. Org. Chem. 23 (1958) 1539 - 1541), durch Benzoinkondensation und anschließende Oxidation aus den entsprechenden Benzaldehyden hergestellt werden. Ausgangsverbindungen, die neu sind, sind beispielsweise 3,3',4,4'-Tetrachlorbenzil und 2,2',4,4'-Tetrachlorbenzil. Benötigt man unsymmetrische Benzile als Ausgangsverbindungen, so können diese außer durch Benzoinkondensation mit anschließender Oxidation beispielsweise durch Acylierungs-/Oxidationsschritte hergestellt und anschließend erfindungsgemäß umgesetzt werden.

Die nachstehenden Beispiele erläutern das Verfahren.

### Beispiel 1

55,8 g (0,2 Mol) 4,4'-Dichlorbenzil und 11 g Tetraphenylphosphoniumbromid werden in 250 g Sulfolan gelöst. In der erhaltenen Lösung werden 46,4 g (0,8 Mol) Kaliumfluorid suspendiert. Anschließend wird 6 h unter Stickstoff bei kräftiger Rührung auf 180 °C erhitzt. Nach dem Abkühlen auf ca. 40 °C wird die so erhaltene Reaktionssuspension abgesaugt und die Mutterlauge fraktioniert. Man erhält so 28,9 g (0,119 Mol, 59 % d. Th.) eines gelben Feststoffes vom Siedepunkt 138 - 143 °C/0,01 Torr. Durch Kristallisation einer Probe aus Ethanol erhält man gelbe Kristalle vom (4,4'-Difluorbenzil) Schmelzpunkt 119 - 121 °C.

### Beispiel 2

11,2 g (0,04 Mol) 4,4'-Dichlorbenzil werden mit 7 g Kaliumfluorid, 20 g Chlornaphthalin, 30 ml Toluol, 4,2 g Tetraphenylphosphoniumbromid und 4,4 g Tetraethylenglycoldimethylether versetzt, das Toluol bis 150 °C/45 Torr abdestilliert und die so erhaltene Reaktionssuspension 18 h auf 205 °C erhitzt. Nach GC-Analyse enthält die Reaktionsmischung 32 % 4,4'-Difluorbenzil, 42 % 4-Fluor-4'-chlorbenzil (MS) und 26 % 4,4'-Dichlorbenzil.

### Beispiel 3

22,4 g (0,08 Mol) 4,4'-Dichlorbenzil werden mit 14 g (0,24 Mol) Kaliumfluorid und 1,0 g 18-Krone-6 in 100 g Sulfolan unter Stickstoff unter Rühren 40 h auf 180 °C erhitzt. Nach GC-Analyse beträgt der Umsatz nach dieser Zeit > 90 %. Erhalten werden 14,3 g (58 mMol, 72 %) 4,4'-Difluorbenzil und 0,7 g (2,6 mMol, 3 %) 4-Fluor-4'-chlorbenzil durch Destillation im Gemisch, das durch Kristallisation getrennt werden kann.

### Beispiel 4

34,8 g (0,1 Mol) 3,3',4,4'-Tetrachlorbenzil werden in 100 g Sulfolan gelöst und unter Stickstoff bei kräftiger Rührung mit 17,5 g (0,3 Mol) Kaliumfluorid und 1,9 g (0,01 Mol) Cäsiumfluorid 6 h auf 180 °C erhitzt. Nach Abkühlen auf ca. 40 °C wird abgesaugt, einmal mit 20 ml Sulfolan gewaschen, die Mutterlauge in 600 ml Wasser gelöst, dreimal mit 300 ml Ether extrahiert, die Etherphase zweimal mit jeweils 100 ml Wasser gewaschen, über Molekularsieb (4 Å) getrocknet und das Lösungsmittel bis 40 °C/20 Torr entfernt. Man erhält so 34,3 g eines dunkelbraunen Feststoffes. Die anschließende Fraktionierung ergibt 17,3 g (0,549 Mol, 55 % d. Th.) 3,3'-Dichlor-4,4'-difluorbenzil als gelben, kristallinen Feststoff vom Siedepunkt 148 °C/0,05 Torr.
- ¹H-NMR [CDCl₃, TMS]:: δ = 7,2 - 7,4 (m, 1H), 7,85 - 7,95 (m, 1H), 8,05 - 8,15 (m, 1H),
- ¹³C-NMR [CDCl₃, TMS]:: δ = 117,58 (d, J = 21,9 Hz), 122,95 (d, J = 17,2 Hz), 129,97 (s), 130,75 (d, J = 9,1 Hz), 132,92 (s), 162,43 (d, J = 260,4 Hz), 190,16 (s)

### Beispiel 5

3,5 g (0,01 Mol) 3,3',4,4'-Tetrachlorbenzil werden mit 1,2 g (0,02 Mol) Kaliumfluorid, 0,5 g Tetraphenylphosphoniumbromid und 1,0 g Benzophenon (interner Standard) in 20 g Sulfolan unter Rühren in Inertgasatmosphäre (Stickstoff) 4 h auf 180 °C erhitzt. Nach GC-Analyse enthält die Reaktionsmischung, bezogen auf eingesetztes 3,3',4,4'-Tetrachlorbenzil, 44 % 3,3'-Dichlor-4,4'-difluorbenzil, 23 % 3,4,4'-Trichlor-3'-fluorbenzil und 13 % 3,3',4,4'-Tetrachlorbenzil.

### Beispiel 6

34,8 g (0,1 Mol) 2,2',4,4'-Tetrachlorbenzil werden in 200 g Sulfolan gelöst und unter Stickstoff mit 50,4 g (0,9 Mol) Kaliumfluorid und 5,6 g (0,1 Mol) Cäsiumfluorid 12 h auf 180 °C erhitzt. Nach dem Abkühlen auf ca. 60 °C wird die Reaktionssuspension abgesaugt und die Mutterlauge fraktioniert. Man erhält so 16,9 g (0,0602 Mol, 60 % d. Th.) 2,2',4,4'-Tetrafluorbenzil als gelblichen Feststoff vom Siedepunkt 132 °C/0,5 Torr.
- ¹HNMR [CDCl₃, TMS]:: δ = 6,75 - 7,2 (m, 2H), 8,0 - 8,2 (m, 1H)
- ¹⁹F-NMR [CDCl₃, CFCl₃]:: δ = - 105 (1F), - 98 (1F)
- ¹³C-NMR [CDCl₃, TMS]:: δ = 104,91 (pseudo t, J = 25,64 Hz), 113,18 (d, J = 22,2 Hz) 118,09 (dd J = 2,2, 13,3 Hz), 133,00 (d, J = 11,1 Hz) 163,98 (dd, J = 13,2, 259,7 Hz) 167,50 (dd, J = 12,4, 261,1 Hz) 188,61 (s)

### Beispiel 7

### Herstellung von 3,3',4,4'-Tetrachlorbenzil

In 125 ml Methanol wurden 241,3 g (1,4 Mol) 3,4-Dichlorbenzaldehyd vorgelegt und unter Argon bei 70 °C 5 g Kaliumcyanid in 7 g Wasser binnen 30 Minuten zugetropft (sofortige Rotfärbung). Bei dieser Temperatur wurde 4 h zum Rückfluß erhitzt. Nach dem Abkühlen wurde eine rote, klare Lösung erhalten. Man entfernte am Rotationsverdampfer 100 g Lösungsmittel (glasartiger, zäher Rückstand: 293,8 g). Der Rückstand wurde mit 600 ml Toluol versetzt und unter Rühren auf 60 °C erhitzt. Anschließend wurde die Suspension bei 45 °C abgesaugt und der Filterkuchen 2 mal mit 100 ml Toluol gewaschen. Nach 24 h wurde Unlösliches abfiltriert und das Filtrat dreimal mit 200 ml 20 %iger Natriumhydrogensulfit-Lösung ausgeschüttet. Die Lösung wurde im Vakuum eingeengt. Es verbleiben 194,1 g Rückstand. 140 g des Rückstandes wurden bei 98 °C vorgelegt. Dazu wurden innerhalb von 2 h 140 ml 70 %ige Salpetersäure zugetropft. Nach Verdünnen bei Siedetemperatur mit 140 g Wasser ließ man langsam abkühlen. Nach Kühlung (0 °C) wurde filtriert und mit 200 g Wasser gewaschen. Man erhielt 144,2 g Rohprodukt, das aus 400 ml Ethanol umgelöst wurde. Man erhielt 54,4 g (0,156 Mol, 31 %) 3,3',4,4'-Tetrachlorbenzil und weitere 24,4 g (0,07 Mol, 14 %) dieser gelben Verbindung in Form von Kristallnadeln durch Einengen der Mutterlauge. Der Schmelzpunkt der Produktfraktionen konnte durch mehrfaches Umkristallisieren (Ethanol) von 165 - 167,5 °C auf 193 - 198 °C verbessert werden.
- MS m/z (%) =: 74 (11), 109 (19), 145 (31), 147 (21), 173 (100), 175 (70), 177 (12), 346 (0.9), 348 (M⊕_{, 1,1)}

### Beispiel 8

### Herstellung von 2,2',4,4'-Tetrachlorbenzil

In 250 ml Methanol wurden 482,5 g (2,8 Mol) 2,4-Dichlorbenzaldehyd vorgelegt und unter Argon bei 68 °C in 1,5 h 10 g Kaliumcyanid in 14 g Wasser zugetropft. Die klare, rote Lösung wurde noch weitere 2 h bei dieser Temperatur gehalten und danach das Methanol bei 95 °C im Vakuum möglichst vollständig entfernt. Der Rückstand (493,1 g) wurde auf 80 °C erwärmt und in 1 l Toluol eingegossen. Die entstandene Lösung wurde 2mal mit je 200 ml 20 %iger Natriumhydrogensulfit-Lösung ausgeschüttelt. Anschließend wurde zur besseren Phasentrennung filtriert und die organische Phase abgetrennt. Nach Entfernen des Toluols im Vakuum verbleiben 440,8 g Rohprodukt als zäher, glasartiger Rückstand.

244,0 g dieses Rückstandes wurden auf 100 °C erhitzt und innerhalb von 2 h 250 ml 70 %ige Salpetersäure zugetropft. Anschließend wurde mit 100 g Wasser verdünnt und nach dem Abkühlen die überstehende Flüssigkeit dekantiert (255,1 g Rohprodukt). Das Rohprodukt wurde durch Umkristallisieren aus Ethanol gereinigt. Man erhielt 145,5 g (0,42 Mol, 54 %) hellgelbes 2,2',4,4'-Tetrachlorbenzil vom Schmelzpunkt 157,5 - 160,5 °C. Aus der Mutterlauge konnten nach Einengen weitere 23,4 g (67 mMol, 9 %) Produkt isoliert werden. Durch mehrfaches Umlösen aus Ethanol wurde reines 2,2',4,4'-Tetrachlorbenzil vom Schmelzpunkt 161 °C erhalten.
- MS m/z (%) =: 50 (4,5), 74, (15,5), 84 (4), 109 (23), 145 (27), 147 (18), 173 (100), 175 (72), 177 (13), 313 (2,3), 348 (M⁺, 0,5).

## Patentansprüche

1. Verfahren zur Herstellung von Benzilen der allgemeinen Formel (1) 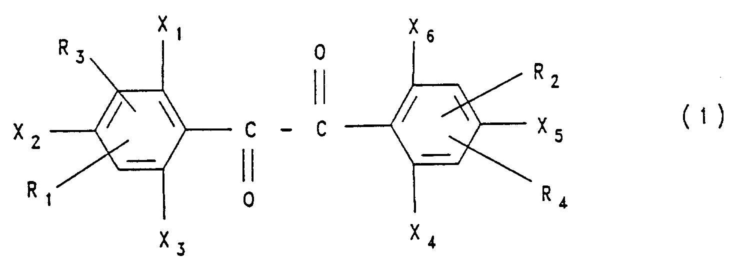 in welcher X₁, X₂, X₃, X₄, X₅, X₆, R₁, R₂, R₃ und R₄ Wasserstoff-, Fluor-, Chlor- oder Bromatome, Alkyl(C₁-C₁₀)gruppen, Phenyl- oder Naphthylgruppen, die durch Fluor-, Chlor- oder Bromatome, Alkyl(C₁-C₄)-, Alkoxy(C₁-C₄)-, Nitro-, Cyano-, -CHO-, -COCl-, -COF-, CF₃-, -SO₂-Alkyl(C₁-C₄)-, -SO₂F-, -SO₂Cl-, -COO-Alkyl(C₁-C₄)-, -CO-N(Alkyl (C₁-C₄))₂-, -CO-Phenyl-, -SO₂-Phenyl-gruppen
substituiert sein können, oder die Elektronendichte herabsetzende Gruppen aus der Reihe Nitro-, Cyano-, -CHO-, -COCl, -COF, -CF₃, -SO₂-Alkyl(C₁-C₄)-, -SO₂F-, -SO₂Cl-, -CO-N(Alkyl(C₁-C₄))₂, -COO-Alkyl(C₁-C₄)-, -CO-Phenyl oder -SO₂-Phenyl bedeuten, mit der Maßgabe, daß mindestens einer der Substituenten X₁ - X₆ ein Fluoratom bedeutet, dadurch gekennzeichnet, daß man 1 Mol eines Benzils der genannten allgemeinen Formel (1), in welcher X₁ - X₆ und R₁, R₂, R₃ und R₄ die genannten Bedeutungen haben, mit der Maßgabe, daß mindestens einer der Substituenten X₁ - X₆ ein Chloratom darstellt, mit 0,8 bis 2,5 Mol Kalium-, Rubidium-, Cäsium- oder Tetraalkyl(C₁-C₁₈)-ammoniumfluorid oder Mischungen davon pro auszutauschendem Chloratom bei Temperaturen von 100 °C bis 280 °C, in Abwesenheit oder in Gegenwart eines Phasentransferkatalysators und in Abwesenheit oder in Gegenwart eines dipolar aprotischen oder unpolaren Lösungsmittels umsetzt.

2. Verfahren nach Anspruch 1, dadurch gekennzeichnet, daß man mit einer Mischung aus Kaliumfluorid und Cäsiumfluorid umsetzt.

3. Verfahren nach mindestens einem der Ansprüche 1 und 2, dadurch gekennzeichnet, daß man bei Temperaturen von 160 °C bis 240 °C umsetzt.

4. Verfahren nach mindestens einem der Ansprüche 1 bis 3, dadurch gekennzeichnet, daß man mit 0,9 bis 1,5 Mol Fluoridsalz pro auszutauschendem Chloratom umsetzt.

5. Verfahren nach mindestens einem der Ansprüche 1 bis 4, dadurch gekennzeichnet, daß man mit 1,0 bis 1,2 Mol Fluoridsalz pro auszutauschendem Chloratom umsetzt.

6. Verfahren nach mindestens einem der Ansprüche 1 bis 5, dadurch gekennzeichnet, daß man als Phasentransferkatalysator quartäre Ammonium- oder Phosphoniumverbindungen, Kronenether oder Dialkylpyridiniumsalze oder Mischungen dieser Verbindungen verwendet.

7. Verfahren nach mindestens einem der Ansprüche 1 bis 6, dadurch gekennzeichnet, daß man als Phasentranferkatalysator Tetraalkyl(C₁-C₁₈)-ammoniumchloride, -bromide oder -fluoride, Tetraalkyl(C₁-C₁₈)-phosphoniumchloride oder -bromide, Tetraphenylphosphoniumchlorid oder -bromid, ((Phenyl)ₘ(alkyl(C₁-C₁₈)ₙ)-phosphoniumchloride oder -bromide, wobei m = 1 bis 3, n = 3 bis 1 und m + n = 4 sind oder 18-Krone-6 verwendet.

8. Verfahren nach mindestens einem der Ansprüche 1 bis 6, dadurch gekennzeichnet, daß man in Gegenwart von 0,01 bis 50 Molprozent eines Phasentransferkatalysators umsetzt.

9. Verfahren nach mindestens einem der Ansprüche 1 bis 5, dadurch gekennzeichnet, daß man als Phasentransferkatalysator Oligo- oder Polyethylenglykoldimethylether in einer Menge von 0,5 bis 200 Massenprozent, bezogen auf die Masse des eingesetzten Fluoridsalzes, einsetzt.

10. Verfahren nach mindestens einem der Ansprüche 1 bis 9, dadurch gekennzeichnet, daß man in Gegenwart von Sulfolan (Tetramethylensulfon), Tetramethylensulfoxid, N,N-Diethylacetamid, N,N-Dimethylacetamid, N,N-Dimethylformamid, N-Methylpyrrolidon, Dimethylsulfoxid, Dimethylsulfon, Diphenylsulfoxid, Diphenylsulfon, Tetramethylharnstoff, Tetra-n-butylharnstoff, 1,3-Dimethylimidazolidin-2-on oder Mischungen daraus als dipolar aprotischen Lösungsmitteln umsetzt.

11. Verfahren nach mindestens einem der Ansprüche 1 bis 10, dadurch gekennzeichnet, daß man in Gegenwart von Chlornaphthalin, Dichlorbenzol, Toluol oder Xylolen als unpolaren Lösungsmitteln umsetzt.

## Claims

1. A process for the preparation of benzils of the formula (1) 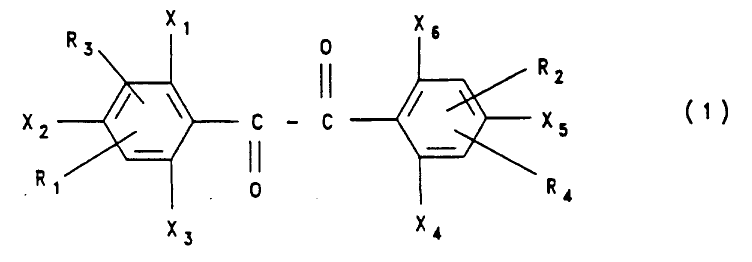 in which X₁, X₂, X₃, X₄, X₅, X₆, R₁, R₂, R₃ and R₄ are hydrogen, fluorine, chlorine or bromine atoms, alkyl(C₁-C₁₀) groups, phenyl or naphthyl groups which may be substituted by fluorine, chlorine or bromine atoms, or by alkyl(C₁-C₄), alkoxy(C₁-C₄), nitro, cyano, -CHO, -COCl, -COF, -CF₃, -SO₂-alkyl(C₁-C₄), -SO₂F, -SO₂Cl, -COO-alkyl(C₁-C₄), -CO-N(alkyl(C₁-C₄))₂, -CO-phenyl or -SO₂-phenyl groups, or groups reducing the electron density, from the group consisting of nitro, cyano, -CHO, -COCl, -COF, -CF₃, -SO₂-alkyl(C₁-C₄), -SO₂F, -SO₂Cl, -CO-N(alkyl(C₁-C₄))₂, -COO-alkyl(C₁-C₄), -CO-phenyl and -SO₂-phenyl, with the proviso that at least one of the substituents X₁-X₆ is a fluorine atom, which comprises reacting 1 mol of a benzil of the abovementioned formula (1) in which X₁-X₆ and R₁, R₂, R₃ and R₄ have the meanings mentioned, with the proviso that at least one of the substituents X₁-X₆ is a chlorine atom, with from 0.8 to 2.5 mol of potassium fluoride, rubidium fluoride, cesium fluoride or tetraalkyl(C₁-C₁₈)-ammonium fluoride or mixtures thereof per chlorine atom to be exchanged at temperatures of from 100°C to 280°C, in the absence or presence of a phase transfer catalyst and in the absence or presence of a dipolar aprotic or nonpolar solvent.

2. The process as claimed in claim 1, which comprises carrying out reaction with a mixture of potassium fluoride and cesium fluoride.

3. The process as claimed in at least one of claims 1 and 2, which comprises carrying out reaction at temperatures of from 160°C to 240°C.

4. The process as claimed in at least one of claims 1 to 3, which comprises carrying out reaction with from 0.9 to 1.5 mol of fluoride salt per chlorine atom to be exchanged.

5. The process as claimed in at least one of claims 1 to 4, which comprises carrying out reaction with from 1.0 to 1.2 mol of fluoride salt per chlorine atom to be exchanged.

6. The process as claimed in at least one of claims 1 to 5, wherein the phase transfer catalyst used is a quaternary ammonium or phosphonium compound, a crown ether or a dialkyl pyridinium salt, or a mixture of these compounds.

7. The process as claimed in at least one of claims 1 to 6, wherein the phase transfer catalyst used is a tetraalkyl(C₁-C₁₈)-ammonium chloride, bromide or fluoride, a tetraalkyl(C₁-C₁₈)-phosphonium chloride or bromide, tetraphenylphosphonium chloride or bromide, a ((phenyl)ₘ(alkyl(C₁-C₁₈)ₙ)-phosphonium chloride or bromide, where m = 1 to 3, n = 3 to 1 and m + n = 4, or 18-crown-6.

8. The process as claimed in at least one of claims 1 to 6, which comprises carrying out reaction in the presence of from 0.01 to 50 mol percent of a phase transfer catalyst.

9. The process as claimed in at least one of claims 1 to 5, wherein the phase transfer catalyst employed is an oligo- or polyethylene glycol dimethyl ether in an amount of from 0.5 to 200 percent by mass, based on the mass of the fluoride salt employed.

10. The process as claimed in at least one of claims 1 to 9, which comprises carrying out reaction in the presence of sulfolane (tetramethylene sulfone), tetramethylene sulfoxide, N,N-diethylacetamide, N,N-dimethylacetamide, N,N-dimethylformamide, N-methylpyrrolidone, dimethyl sulfoxide, dimethyl sulfone, diphenyl sulfoxide, diphenyl sulfone, tetramethyl urea, tetra-n-butyl urea, 1,3-dimethylimidazolidin-2-one or a mixture thereof as dipolar aprotic solvent.

11. The process as claimed in at least one of claims 1 to 10, which comprises carrying out reaction in the presence of chloronaphthalene, dichlorobenzene, toluene or a xylene as nonpolar solvent.

## Revendications

1. Procédé de préparation de benziles de formule générale (1) 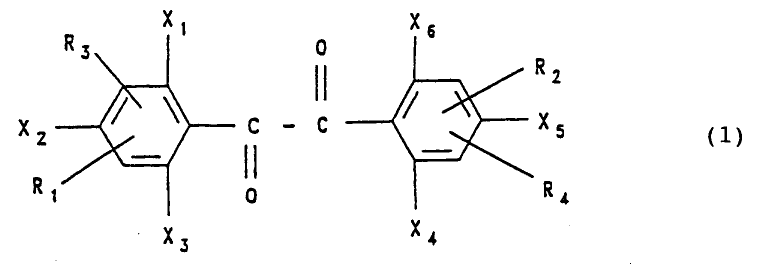 dans laquelle X₁, X₂, X₃, X₄, X₅, X₆, R₁, R₂, R₃ et R₄ représentent des atomes d'hydrogène, de fluor, de chlore ou de brome, des groupes alkyle en C₁-C₁₀, des groupes phényle ou naphtyle, qui peuvent être substitués par des atomes de fluor, de chlore ou de brome, des groupes alkyle en C₁-C₄, alkoxy en C₁-C₄, nitro, cyano, -CHO, -COCl, -COF, CF₃-, -SO₂-alkyle en C₁-C₄, -SO₂F, -SO₂-Cl, -COO-alkyle en C₁-C₄, -CO-N-(alkyle en C₁-C₄)₂, -CO-phényle, -SO₂-phényle,
ou des groupes réduisant la concentration d'électrons pris parmi les groupes nitro, cyano, -CHO, -COCl, -COF, -CF₃, -SO₂-alkyle en C₁-C₄, -SO₂F, -SO₂Cl, -CON-(alkyle en C₁-C₄)₂-, -COO-alkyle en C₁-C₄, -CO-phényle- ou -SO₂-phényle, à la condition qu'au moins un des substituants X₁ à X₆ représente un atome de fluor, caractérisé en ce qu'on fait réagir 1 mole d'un benzile de formule (1) précitée, dans laquelle X₁ à X₆ et R₁, R₂, R₃ et R₄ possèdent les significations citées, à la condition qu'au moins un des substituants X₁ à X₆ représente un atome de chlore, avec une quantité de 0,8 à 2,5 moles de fluorure de potassium, de rubidium, de césium ou de tétra(alkyl en C₁-C₁₈)ammonium ou leurs mélanges par atome de chlore à échanger, à une température de 100°C à 280°C, en l'absence ou en présence d'un catalyseur de transfert de phase et en l'absence ou en présence d'un solvant aprotique dipolaire ou non polaire.

2. Procédé selon la revendication 1, caractérisé en ce qu'on met en oeuvre la réaction avec un mélange de fluorure de potassium et de fluorure de césium.

3. Procédé selon au moins l'une des revendications 1 et 2, caractérisé en ce qu'on met en oeuvre la réaction à une température de 160°C à 240°C.

4. Procédé selon au moins l'une des revendications 1 à 3, caractérisé en ce qu'on met en oeuvre la réaction avec 0,9 à 1,5 mole de sel fluorure par atome de chlore à échanger.

5. Procédé selon au moins l'une des revendications 1 à 4, caractérisé en ce qu'on met en oeuvre la réaction avec 1,0 à 1,2 mole de sel fluorure par atome de chlore à échanger.

6. Procédé selon au moins l'une des revendications 1 à 5, caractérisé en ce qu'on utilise comme catalyseur de transfert de phase des composés de phosphonium ou d'ammonium quaternaires, des éthers couronne ou des sels de dialkylpyridinium ou des mélanges de ces composés.

7. Procédé selon au moins l'une des revendications 1 à 6, caractérisé en ce qu'on utilise comme catalyseur de transfert de phase des chlorures, des bromures ou des fluorures de tétra(alkyl en C₁-C₁₈)ammmonium, des chlorures ou bromures de tétra(alkyl en C₁-C₁₈)-phosphonium, le chlorure ou le bromure de tétraphénylphosphonium, des chlorures ou des bromures de ((phényl)ₘ(alkyl en C₁-C₁₈)ₙ)-phosphonium, où m = 1 à 3, n = 3 à 1, n + m = 4 ou le 18-couronne-6.

8. Procédé selon au moins l'une des revendications 1 à 6, caractérisé en ce qu'on met en oeuvre la réaction en présence de 0,01 à 50% molaires d'un catalyseur de transfert de phase.

9. Procédé selon au moins l'une des revendications 1 à 5, caractérisé en ce qu'on utilise comme catalyseur de transfert de phase des éthers diméthyliques d'oligo- ou de polyéthylène glycol en une quantité de 0,5 à 200% massiques, par rapport à la masse du sel fluorure utilisé.

10. Procédé selon au moins l'une des revendications 1 à 9, caractérisé en ce qu'on met en oeuvre la réaction en présence de la sulfolane (tétraméthylsulfone), du tétraméthylsulfoxyde, du N,N-diéthylacétamide, du N,N-diméthylacétamide, du N,N-diméthylformamide, de la N-méthylpyrrolidone, du diméthylsulfoxyde, de la diméthylsulfone, du diphénylsulfoxyde, de la diphénylsulfone, de la tétraméthylurée, de la tétra-n-butylurée, de la 1,3-diméthylimidazolidin-2-one (DMI) ou leurs mélanges en tant que solvants aprotiques dipolaires.

11. Procédé selon au moins l'une des revendications 1 à 10, caractérisé en ce qu'on met en oeuvre la réaction en présence du chloronaphtalène, du dichlorobenzène, du toluène ou du xylène en tant que solvants non polaires.
